# FASCICULE DE BREVET EUROPEEN

(11) **EP 0 403 333 B1**
(45) Date de publication et mention de la délivrance du brevet: **06.10.1999**
(21) Numéro de dépôt: 90401520.3
(22) Date de dépôt: 05.06.1990
(51) Int. Cl.: C12Q 1/70, C07H 21/04, C12Q 1/68, A61K 39/21, G01N 33/569, A61K 39/42, A61K 31/70, C12N 15/49

(54) **Séquences nucléotidiques issues du génome des rétrovirus du typ hiv-1, et leurs applications notamment pour l'amplification des génomes de ces rétrovirus et pour le diagnostic in-vitro des infections dues à ces virus**
Nukleotid-Sequenzen des Genoms von Retroviren vom Typ HIV-1, HIV-2 und SIV, ihre Verwendungen zur Amplifikation dieser Genome und in-vitro-Diagnose dieser viralen Infektionen
Nucleotide sequences of retroviral genomes of types HIV-I, HIV-2 and SIV, their uses for the amplification of these genomes and diagnosis in vitro of these viral infections

(30) Priorité: 02.06.1989 FR 8907354; 20.09.1989 FR 8912371
(43) Date de publication de la demande: 19.12.1990
(60) Demande divisionnaire: 97110543.2
(73) Titulaire: INSTITUT PASTEUR, 75724 Paris Cédex 15 (FR); INSTITUT NATIONAL DE LA SANTE ET DE LA RECHERCHE MEDICALE (INSERM), 75654 Paris Cédex 13 (FR)
(72) Inventeur: Moncany, Maurice, F-75019 Paris (FR); Montagnier, Luc, F-92350 Le Plessis-Robinson (FR)
(74) Mandataire: Desaix, Anne

(56) Documents cités:
- EP-A- 0 229 701
- EP-A- 0 269 445
- EP-A- 0 269 520
- EP-A- 0 272 098
- WO-A-87/07300
- WO-A-87/07906
- WO-A-88/05440
- THE LANCET, vol. 2, 16 septembre 1989, pages 637-639, The Lancet Ltd; C.R. HORSBURGH, Jr.: "Duration of human immunodeficiency virus infection before detection of antibody"
- SCIENCE, vol. 239, 15 janvier 1988, pages 295-297; C.-H. OU et al.: "DNA amplification for direct detection of HIV-1 in DNA of peripheral blood mononuclear cells"
- THE JOURNAL OF INFECTIOUS DISEASES, vol. 158, no. 6, décembre 1988, pages 1170-1176; M. RAYFIELD et al.: "Mixed human immunodeficiency virus (HIV) infection in an individual: Demonstration of both HIV type 1 and type 2 proviral sequences by using polymerase chain reaction"
- PROC. NATL. ACAD. SCI. USA, vol. 86, avril 1989, pages 2423-2427; D.J. KEMP et al.: "Colorimetric detection of specific DNA segments amplified by polymerase chain reactions"
- CELL, vol. 58, no. 5, 8 septembre 1989, pages 901-910, Cell Press; A. MEYERHANS et al.: "Temporal fluctuations in HIV Quasispecies in vivo are not reflected by sequential HIV isolations"

## Description

La présente invention est relative à des séquences oligonucléotidiques utilisables pour la mise en oeuvre de techniques d'amplification de séquences nucléiques spécifiques de rétrovirus d'immunodéficience humaine du type HIV ou de rétrovirus d'immunodéficience du singe du type SIV.

L'invention concerne en particulier l'application de ces séquences à des méthodes de diagnostic in vitro chez l'homme de l'infection d'un individu par un rétrovirus du type HIV (actuellement HIV-1 et/ou HIV-2).

L'isolement et la caractérisation de rétrovirus regroupés sous les désignations HIV-1 et HIV-2 ont été décrits dans les demandes de brevet européen n^{o} 85/905.513.9 et n^{o} 87/400.151.4 respectivement. Ces rétrovirus ont été isolés chez plusieurs malades présentant des symptômes d'une lymphadénopathie ou d'un Syndrome d'Immunodeficience Acquise (SIDA).

Les rétrovirus du type HIV-2 comme les rétrovirus du type HIV-1, se caractérisent par un tropisme pour les lymphocytes T4 humains et par un effet cytopathogène à l'égard de ces lymphocytes lorsqu'ils s'y multiplient, pour alors causer entre autres des polyadénopathies généralisées et persistantes, ou un SIDA.

Un autre rétrovirus, dénommé SIV-1, cette dénomination remplaçant la dénomination antérieurement connue STLV-III, a été isolé chez le singe macaque rhésus (M.D. DANIEL et al. Science, 228, 1201 (1985) ; N.L. LETWIN et al, Science, 230, 71 (1985) sous l'appellation "STLV-IIImac").

Un autre rétrovirus, désigné "STLV-III_{AGM}", (ou SIV_{AGM}) a été isolé chez des singes verts sauvages. Mais contrairement aux virus présents chez le singe macaque rhésus, la présence de STLV-III_{AGM} ne semble pas induire une maladie du type SIDA chez le singe vert d'Afrique.

Pour la commodité du langage, ces virus ne seront plus désignés dans ce qui suit que par l'expression SIV (l'expression SIV est l'abréviation anglaise de "Simian Immunodeficency Virus" (Virus d'immunodéficience du singe) éventuellement suivie d'une abréviation désignant l'espèce de singe dont ils sont issus par exemple "MAC" pour le macaque" ou "AGM" pour le singe vert d'Afrique (abréviation de "African Green Monkey").

Une souche du rétrovirus SIV-1Mac à été déposée à la C.N.C.M le 7 février 1986 sous le n^{o} I-521.

La poursuite de l'étude des rétrovirus HIV-1 et HIV-2 a également conduit à l'obtention de séquences d'ADN complémentaires (ADNc) des ARN de leur génome. La séquence nucléotidique complète d'un ADNc d'un rétrovirus représentatif de la classe HIV-2 (HIV-2 ROD) a été déposée le 21/02/1986 à la C.N.C.M. sous le n^{o} I-522, sous le nom de référence LAV-2 ROD.

De même, la séquence nucléotidique complète d'un ADNc d'un rétrovirus représentatif de la classe HIV-1 est décrite par WAIN HOBSON, SONIGO, COLE, DANOS et ALIZON dans CEll (janvier 1985).

Egalement pour la commodité du langage, les virus du type HIV-1 et HIV-2 seront parfois désignés dans ce qui suit par l'expression HIV.

Les méthodes de diagnostic in vitro des infections par des virus du type HIV-1 ou HIV-2 existant actuellement, font appel à la détection d'anticorps ANTI-HIV-1 ou anti-HIV-2 éventuellement présents dans un prélèvement biologique (biopsie) ou dans un fluide biologique, par exemple dans un sérum obtenu, à partir du patient à l'étude, par mise en contact de ce fluide biologique avec des extraits ou antigènes d'HIV-1 ou d'HIV-2, dans des conditions permettant la production d'une réaction immunologique éventuelle de ces ces extraits ou antigènes avec ces anticorps.

De telles méthodes de diagnostic risquent d'être faussement négatives, en particulier dans le cas d'une infection récente d'un individu par les virus du type HIV.

Les techniques d'amplification génique sont d'un appoint considérable pour la mise au point de méthodes de diagnostic in vitro particulièrement sensibles de maladies virales. Parmi ces techniques d'amplification génique, on peut citer la technique PCR (Polymérase Chain Reaction) telle que décrite dans les demandes de brevet européen n^{o} 86/302.298.4 du 27/03/1986 et n^{o} 87/300.203.4 du 09/01/1987, ou encore la technique dite "Qβreplicase" décrite dans Biotechnology, vol.6, page 1197 (octobre 1988) et celle procédant à l'aide d'une ARN polymérase (T7RNA polymérase) décrite dans la demande de brevet international n^{o} WO89/01050. Ces techniques permettent d'améliorer la sensibilité de détection des acides nucléiques des virus, et nécessitent l'utilisation d'amorces de synthèse spécifiques.

Pour la recherche des virus du type HIV, le choix des amorces est problématique. En effet, du fait de la grande variabilité des séquences de nucléotides du génome viral, une amorce conforme à la séquence connue d'un isolat donné d'un virus du type HIV peut faillir à l'amplification de certains variants viraux du type HIV. D'autre part, même si une amorce est choisie dans une région conservée du génome d'un virus HIV à un autre, son "bon fonctionnement" n'est pas pour autant assuré et peut donner lieu à de mauvais rendements d'amplification.

La présente invention fournit précisément des amorces oligonucléotidiques permettant, entre autres, l'amplification, notamment à des fins diagnostiques, du génome de tous virus du type HIV et SIV, avec des rendements considérés comme maximum dans l'état actuel de la technique et surtout évitant la présence de nombreuses bandes aspécifiques.

Les amorces de la présente invention sont à la fois spécifiques des virus du groupe HIV-1 et/ou des virus des groupes HIV-2 et SIV, et sont insensibles aux variations du génome de ces virus.

La présente invention a pour objet des amorces oligonucléotidiques, d'environ 15 à 30 nucléotides, utilisables pour l'amplification génomique des virus du type HIV-1 et/ou du type HIV-2 et SIV.

L'invention concerne toute séquence nucléotidique caractérisée en ce que sa séquence :
- soit est choisie parmi celles qui sont contenues dans l'une des séquences nucléotidiques comprises dans les gènes gag, vpr et pol des virus HIV-1 Bru, HIV-1 Mal, HIV-1 Eli, HIV-2 ROD et SIV MAC, ou dans les gènes nef2, vif2 et vpx des virus HIV-2 ROD, et SIV MAC, ou dans les gènes env, nef1, vif1 et vpr des virus HIV-1 Bru, HIV-1 Mal, et HIV-1 Eli, et plus particulièrement parmi celles qui sont contenues dans les enchaînements nucléotidiques définis ci-après,
- soit (notamment pour les séquences les plus longues) contient l'une des séquences nucléotidiques susdites issues de HIV-1 Bru, ou HIV-1 Mal, ou HIV-1 Eli ou HIV-2 ROD ou SIVMac , ou contient une séquence nucléotidique complémentaire de l'une de ces dernières séquences, étant entendu que les nucléotides supplémentaires éventuels qui "débordent" la séquence nucléotidique du genre en question, du côté des extrémités 3' ou 5', coïncident de préférence avec ceux qui se trouvent placés en deçà des extrémités 5' ou 3' correspondant au sein même de la séquence complète des virus du type HIV-1, HIV-2 ou de SIV MAC, sus-mentionnés,
- soit, si cette séquence nucléotidique n'est pas identique à l'une des séquences nucléotidiques susdites, ou n'est pas complémentaire de l'une de ces séquences, est néanmoins susceptible de s'hybrider avec une séquence nucléotidique issue des virus HIV-1 Bru,HIV-1 Mal, HIV-1 Eli, et/ou avec une séquence nucléotidique issue du virus HIV-2 ROD ou SIV MAC sus-mentionnée. L'hybridation peut s'effectuer à une température de 60°C ± 1°C (de préférence 60°C ± 0,5°C), préconisée pour un optimum de rendement.

La numérotation des nucléotides mentionnés ci-dessous correspond à celle utilisée dans le manuel de référence "Human Retrovirus and AIDS-1989" édité par le "Los Alamos National Laboratory- New Mexico - USA".

(Les séquences des virus HIV-1 Mal, HIV-1 Eli ont été décrites par MONTAGNIER, SONIGO, WAIN-HOBSON et ALIZON dans la demande de brevet européen n° 86.401380 du 23/06/86).

Les séquences de l'invention sont synthétisées sur synthétiseur commercialisé par Applied Biosystems (méthode phosphoro-amidites), ou sur tout autre appareil utilisant une méthode semblable.

L'invention concerne plus particulièrement les séquences oligonucléotidiques caractérisées par les enchaînements nucléotidiques suivants (représentés dans le sens 5' → 3'; les initiales "S" et "AS" indiquent si l'oligonucléotide est sens ou antisens, c'est-à-dire si l'oligonucléotide est orienté respectivement dans le sens 5'○→ 3' ou dans le sens 3' ○→ 5') :
1°) séquences communes aux génomes des virus HIV-1, HIV-2 et SIV (les séries de chiffres espacées d'un trait indiquent la position des nucléotides sur les génomes correspondant respectivement aux virus HIV-1 Bru, HIV-1 Mal, HIV-1 Eli, HIV-2 ROD et SIV) :
   . séquences spécifiques du gène gag du génome des virus sus-mentionnés (gène codant pour un groupe d'antigènes spécifiques du nucléoïde de ces virus).
      Certaines variantes peuvent être apportées sur certaines positions des séquences nucléotidiques indiquées ci-dessous, sans que les propriétés d'hybridation de ces séquences nucléotidiques avec les gènes des virus du type HIV et/ou SIV soient affectées. Les séquences nucléotidiques comportant ces variantes sont représentées en-dessous des séquences nucléotidiques initiales dont elles dérivent par remplacement d'une ou plusieurs bases. Les bases modifiées par rapport à celles des séquences nucléotidiques initiales sont indiquées en toute lettre à la verticale des positions correspondant aux bases qui ont été remplacées dans ces séquences initiales ; tandis que les bases des séquences initiales qui n'ont pas été remplacées dans les séquences comportant ces variantes sont indiquées à l'aide de pointillés.
      La synthèse des amorces se fait en utilisant toutes les variantes simultanément. C'est le mélange de toutes les variantes pour une séquence donnée qui est utilisé dans les tests. 
MMy1 :   TGG CGC CCG AAC AGG GAC
         ... ... .T. ... ... ...
         S, 636-653, 635-652, 636-653, 859-876, 834-851
 
MMy2 :    GGC CAG GGG GAA AGA AAA A
          ... .C. .C. ... ... ... .
          ... ... .A. ... ... ... .
         S,854-872,864-888,848-872,1160-1184, 1124-1148
 
MMy3 :    TGC CCA TAC AAA ATG TTT TA
          ... ... C.. T.T ... ... ..
       AS,900-881,916-897,900-881,1212-1193,1176-1157
 
MMy4 : TGC ATG GCT GCT TGA TG
... ..A ... ..C ..G ..
AS,1385-1369,1419-1403,1385-1369,1703-1687,1667-1651
 
MMy4B : CTT TGC ATG GCT GCT TGA TG
..C ... ..A ... ..C ..G ..
AS, 1388-1369, 1421-1403, 1388-1369, 1706-1687,
1670-1651,
 
MMy4Bbis : CAT CAA GCA GCC ATG CAA AG
..C ..G ... ..T ... ..G ..
S, 1369-1388, 1403-1421, 1369-1388,
1687-1706, 1651-1670,
 
MMy28 : AGG GCT GTT GGA AAT GTG G
... ... ... ... ..G ... .
S, 2021-2039, 2055-2073, 2024-2042, 2329-2349,
2299-2318,
 
MMy28bis : CCA CAT TTC CAG CAT CCC T
... ... ... ... ..G ... .
... ... ... ... ..C ... .
AS, 2039-2021, 2073-2055, 2042-2024, 2349-2329,
2318-2299
   . séquences spécifiques du gène vpr 
MMy18 : GAT AGA TGG AAC AAG CCC CAG
S, 5590-5610, 5585-5605, 5554-5574, 6233-6296,
6147-6170,
 
MMy19 : TCC ATT TCT TGC TCT CCT CTG T
AS, 5870-5849, 5865-5844, 5834-5813,
6551-6531, 6454-6431,
   . séquences spécifiques du gène pol : 
MMy29 : TAA AGC CAG GAA TGG ATG GCC CAA
... ... ... ... ... ... .A. ...
S, 2620-2643, 2615-2638, 2584-2607, 2971-2994,
2887-3010
 
MMy29bis : TTG GGC CAT CCA TTC CTG GCT TTA
           ... .T. ... ... ... ... ... ...
        AS, 2643-2620, 2638-2615, 2607-2584, 2994-2971,
           3010-2887,
 
MMy30 : TGG ACT GTC AAT GAC ATA CAG AA
... ... ... ... ..T ... ... ..
S, 3339-3361, 3334-3356, 3303-3325, 3690-3712,
3606-3628,
 
MMy30bis : TTC TGT ATG TCA TTG ACA GTC CA
... ... ... ... ... ..T ... ..
AS, 3361-3339, 3356-3334, 3325-3303, 3712-3690,
3628-3606,
 
MMy31 : CAT GGG TAC CAG CAC ACA AAG G
S, 4186-4207, 4181-4202, 4150-4171, 4534-4555,
4450-4471,
 
MMy31bis : CCT TTG TGT GCT GGT ACC CAT G
AS, 4207-4186, 4202-4181, 4171-4150, 4555-4534,
4471-4450,
 
MMy32 : TGG AAA GGT GAA GGG GCA GT
... ... ... ... ..A ... ..
S, 4992-5011, 4987-5006, 4956-4975, 5340-5359,
5256-5275,
 
MMy32bis : ACT GCC CCT TCA CCT TTC CA
... ... ... ..T ... ... ..
... ... ... ..C ... ... ..
AS, 5011-4992, 5006-4987, 4975-4956, 5359-5340,
5275-5256
2°) séquences communes aux génomes des virus HIV-2 et SIV (les séries de chiffres espacées d'un trait indiquent la position des nucléotides sur les génomes correspondant respectivement aux virus HIV-2 ROD, et SIV-MAC).
   . séquences spécifiques du gène nef2 (codant pour un facteur négatif de 27 kD) 
MMy12 : AGA GAC TCT TGC GGG CGC GTG
S, 9165-9185, 9139-9159,
 
MMy13 : ATA TAC TTA GAA AAG GAA GAA GG
S, 9542-9564, 9516-9538,
 
MMy13bis :   CCT TCT TCC TTT TCT AAG TAT AT
             AS, 9564-9542, 9538-9516,
 
MMy14 : AGC TGA GAC AGC AGG GAC TTT CCA
AS, 9956-9933, 9893-9870,
   . séquences spécifiques du gène vif2 (codant pour un facteur d'infectiosité de 23 kD) 
MMy20 : TAT GGA GGA GGA AAA GAG ATG GAT AGT
S, 5424-5450, 5340-5366,
 
MMy21 : TAG CAC TTA TTT CCC TTG CTT T
S, 5754-5775, 5670-5691,
 
MMy21bis : AAA GCA AGG GAA ATA AGT GCT A
AS, 5775-5754, 5691-5670,
 
MMy22 : CCC TTG TTC ATC ATG CCA GTA T
AS, 6082-6061, 5995-5974,
   . séquences spécifiques du gène vpx (codant pour une protéine de 12 kD) 
MMy23 : ATG TCA GAT CCC AGG GAG A
S, 5900-5918, 5813-5831,
 
MMy24 : CCT GGA GGG GGA GGA GGA GGA
AS, 6228-6208, 6141-6121,
3°) Séquences communes aux génomes des virus HIV-1 Bru, HIV-1 Mal, et HIV-1 Eli (les séries de chiffres espacées d'un trait indiquent la position des nucléotides sur les génomes correspondant respectivement aux virus HIV-1 Bru, HIV-1 MAl et HIV-1 Eli).
   . séquences spécifiques du gène env (codant pour les protéines d'enveloppe) 
MMy5 : CCA ATT CCC ATA CAT TAT TGT GCC CC
S,6905-6930,6903-6928,6860-6885
 
MMy5bis : GGG GCA CAA TAA TGT ATG GGA ATT GG
AS, 6930-6905, 6928-6903, 6885-6860,
 
MMy6 : AAT GGC AGT CTA GCA GAA GAA GA
S,7055-7077,7053-7075,7010-7032
 
MMy7 : ATC CTC AGG AGG GGA CCC AGA AAT T
S, 7360-7384, 7349-7373,7306-7330
 
MMy7bis : AAT TTC TGG GTC CCC TCC TGA GGA T
AS, 7384-7360,7373-7349,7330-7306
 
MMy8 : GTG CTT CCT GCT GCT CCC AAG AAC CC
AS, 7857-7832,7846-7821,7800-7775
 
MMy8bis : GGG TTC TTG GGA GCA GCA GGA AGC AC
S, 7832-7857, 7821-7846, 7775-7800,
 
MMy9 : ATG GGT GGC AAG TGG TCA AAA AGT AG
       ... ... ... ..A ... ... ... ... ..
       S,8844-8869, 8836-8861, 8787-8812,
 
MMy9bis : CTA CTT TTT GAC CAC TTG CCA CCC AT
AS, 8869-8844, 8861-8836, 8812-8787,
 
MMy78 : TAT TAA CAA GAG ATG GTG G
S, 7629-7647, 7612-7630, 7572-7590,
 
MMy89 : CCA GCA AGA AAA GAA TGA A
S, 8224-8242, 8213-8231, 8167-8185,
 
MMy89bis : TTC ATT CTT TTC TTG CTG G
AS, 8242-8224, 8231-8213, 8185-8167,
   . séquences spécifiques du gène nef1 
MMy10 : AAA AGA AAA GGG GGG ACT GGA
S, 9116-9136, 9117-9137, 9062-9082,
 
MMy10bis : TCC AGT CCC CCC TTT TCT TTT
           AS,9136-9116, 9137-9117, 9082-9062,
 
MMy11 : AAA GTC CCC AGC GGA AAG TCC C
AS,9503-9483, 9505-9484, 9449-9428,
   . séquences spécifiques du gène vif 1 
MMy15 : GAT TAT GGA AAA CAG ATG GCA GGT GAT
S, 5073-5099, 5068-5094, 5037-5063,
 
MMy16 : GCA GAC CAA CTA ATT CAT CTG TA
S, 5383-5405, 5378-5400, 5347-5369,
 
MMy16bis : TAC AGA TGA ATT AGT TGG TCT GC
AS, 5405-5383, 5400-5378, 5369-5347,
 
MMy17 : CTT AAG CTC CTC TAA AAG CTC TA
AS, 5675-5653, 5670-5648, 5639-5617,
   . séquences spécifiques du gène vpu

MMy25 : GTA AGT AGT ACA TGT AAT GCA ACC T
S, 6081-6105, 6076-6100, 6045-6069,

MMy26 : AGC AGA AGA CAG TGG CCA TGA GAG
S, 6240-6263, 6238-6261, 6207-6230,

MMy27 : ACT ACA GAT CAT CAA TAT CCC AA
AS, 6343-6321, 6338-6316, 6307-6285,

L'invention a également pour objet les séquences (ou amorces) possédant une structure nucléotidique complémentaire de celles des amorces définies ci-dessus.

Elle concerne également les séquences nucléotidiques présentant certaines mutations par rapport à celles définies ci-dessus sans que les propriétés d'hybridation, telles que définies ci-dessus, de ces séquences soient modifiées. Le pourcentage de nucléotides différents de ceux constituant les séquences décrites ci-dessus, sans pour autant affecter les propriétés d'hybridation des séquences de l'invention, peut atteindre 40 %.

D'une manière générale, dans le cas d'une amorce (primer) sens (S), un plus grand nombre de mutations seront tolérées du côté 5' que du côté 3' de l'amorce, le côté 3' devant s'hybrider parfaitement avec un brin déterminé d'une séquence nucléique pour permettre l'amplification de cette séquence. Dans le cas d'une amorce anti-sens(AS), la tolérance est permise du côté 3'.

L'invention a également pour objet les amorces telles que définies ci-dessus et comportant une conservation d'au moins 5 bases de chaque côté, la partie médiane comportant des modifications, sans que les propriétés d'hybridation ci-dessus soient modifiées.

Une des caractéristiques des amorces oligonucléotidiques de l'invention est de donner une bande d'amplification nette, dépourvue généralement de bandes aspécifiques lorsque les indications techniques d'utilisation décrites dans la présente invention sont mises en oeuvre. Ce fait est dû à la longueur des amorces pouvant atteindre 27 bases ce qui accroît la spécificité d'hybridation, ainsi qu'aux conditions d'utilisation drastiques qui permettent d'éliminer les associations parasites. La spécificité pour chaque type de virus est fonction, outre du pourcentage d'homologie avec la matrice de référence, de la longueur des amorces, qui peuvent atteindre, pour un rendement acceptable, jusqu'à 40 bases.

L'invention s'étend également aux amorces telles que décrites ci-dessus liées au niveau de leur extrémité 5' à un promoteur pour la mise en oeuvre d'une méthode d'amplification génomique par synthèse de multiple copies d'ADN ou d'ARN telle que décrite dans la demande de brevet européenne n^{o} 88/307.102.9 du 01/08/1988.

L'invention a notamment pour objet l'utilisation des amorces décrites ci-dessus pour la mise en oeuvre d'un procédé d'amplification génique de séquences nucléiques de virus du type HIV-1 et/ou HIV-2, et/ou SIV, ce procédé étant applicable au diagnostic in vitro de l'infection potentielle d'un individu par un virus du type HIV-1 et/ou HIV-2 ou d'un animal par au moins l'un des trois virus (HIV-1, HIV-2, SIV).

Cette méthode de diagnostic in vitro de l'invention est réalisée à partir d'un échantillon biologique (par exemple un fluide biologique tel que le sérum, les lymphocytes du sang circulant) obtenu à partir d'un patient à l'étude, et comprend principalement les étapes suivantes :
- une étape d'extraction de l'acide nucléique à détecter appartenant au génome du virus du type HIV-1 et/ou HIV-2 et/ou SIV éventuellement présent dans l'échantillon biologique sus-mentionné, et,le cas échéant, une étape de traitement à l'aide d'une transcriptase inverse dudit acide nucléique si ce dernier est sous forme d'ARN afin d'obtenir un acide nucléique double brin (cette dernière étape étant encore désignée ci-dessous par étape de rétro-transcription de l'ARN viral),
- un cycle comprenant les étapes suivantes :
   . dénaturation de l'acide nucléique double brin à détecter , ce qui conduit à la formation d'un acide nucléique simple brin,
   . hybridation de chacun des brins d'acide nucléique, obtenus lors de l'étape de dénaturation précédente, avec au moins une amorce selon l'invention, par mise en contact des brins sus-mentionnés avec au moins un couple d'amorces selon l'invention dans les conditions d'hybridation définies ci-dessous,
   . formation à partir des amorces des ADN complémentaires aux brins sur lesquels elles sont hybridées en présence d'un agent de polymérisation (ADN polymérase) et de quatre nucléosides triphosphate (dNTP) différents, ce qui conduit à la formation d'un plus grand nombre d'acides nucléiques double brin à détecter qu'à l'étape de dénaturation précédente,
   ce cycle étant répété un nombre de fois déterminé pour obtenir ladite séquence nucléique à détecter éventuellement présente dans l'échantillon biologique dans une proportion suffisante pour permettre sa détection,
- une étape de détection de la présence éventuelle de l'acide nucléique appartenant au génome du virus du type HIV-1 et/ou HIV-2 et/ou SIV dans l'échantillon biologique.

L'étape d'hybridation décrite ci-dessus est avantageusement réalisée à 60°C pendant 1 minute 30 secondes dans le tampon "10 X buffer" dont la composition (en concentration finale d'utilisation) est indiquée ci-dessous.

La méthode de diagnostic in-vitro de l'invention peut être réalisée soit à partir de l'ARN viral, soit à partir de l'ADN complémentaire, épisomial ou intégré.

En effet, les génomes des virus HIV et SIV se présentent sous forme d'ARN ou d'ADN en fonction de la localisation du virus dans l'organisme.

Lorsque le virus est situé à l'intérieur des cellules de l'organisme, notamment à l'intérieur des cellules sanguines, son ARN est recopié en ADN par une transcriptase inverse. En revanche, le génome des virus du type HIV en milieu extracellulaire, notamment dans le sang, demeure sous forme d'ARN.

L'étape d'extraction selon l'invention de l'ADN viral contenu dans les cellules de l'échantillon biologique préconisée par les inventeurs - outre la méthode classique au phénol chloroforme - présente les étapes suivantes :
. suspension du culot cellulaire dans 0,5 ml d'eau pyrolisée dans un Potter gros piston,
. broyage des cellules dit par "aller et retour",
. adjonction Triton X100 pour 1 concentration finale de 0,1 %,
. dénaturation à la chaleur durant 15 à 25 minutes à 100°C,
. centrifugation courte pour n'éliminer que les débris cellulaires,
. précipitation de l'ADN durant la nuit à -20°C par adjonction de 2,5 volumes d'éthanol absolu et de 10 % du volume final d'acétate de sodium 3 Molaires. L'ADN est ensuite récupéré puis resuspendu dans l'eau pyrolysée après avoir été lavé 2 fois par de l'éthanol à 70°. Il est à noter que cette méthode permet la précipitation conjointe des ADN et des ARN, ce qui autorise la détection du message génomique des virus de types HIV ou SIV par utilisation de la méthode dite "PCR directe ADN" ou par celle dite de "PCR-ARN".

L'étape d'extraction de l'ARN viral est généralement effectuée de manière classique connue de l'homme de l'Art.

Après extraction de l'ARN, une étape supplémentaire de transformation de l'ARN, monobrin en ADN double brin est nécessaire à effectuer lorsque le diagnostic in vitro de l'invention est réalisé à partir d'échantillons biologiques contenant les virus du type HIV-1 et/ou HIV-2 et/ou SIV dont les génomes sont sous forme d'ARN.

Cette transformation de l'ARN en ADN est réalisée par traitement de l'ARN obtenu après extraction de l'échantillon biologique, notamment du sérum, dans un milieu approprié à l'aide d'une transcriptase inverse.

L'invention a plus particulièrement entre autres pour objet une méthode de diagnostic in vitro telle que définie ci-dessus, dans laquelle l'étape de rétro-transcription de l'ARN viral est réalisée de la manière suivante :
- 10 µg d'ARN extrait resuspendu dans de l'eau est mis en présence du couple d'amorces à la concentration de 40 µM chacun, dans un volume final de 40 µl. L'ensemble est dénaturé à 100°C durant 10 minutes puis plongé dans de l'eau glacée,
- l'on rajoute 10 µl du mélange suivant : 5 µl du tampon "10 X buffer" décrit ci-dessous + 1 unité de reverse-transcriptase (d'AMV (Avian Myeloblastosis Virus) ou de MuMLV (Moloney Leukemia Virus)) + 1 unité de Taq-polymérase + 1 µl du mélange des 4 dNTP à 25 mM chacun + de l'eau Q.S.P. 10 µl. Le volume final est donc de 50 µl.

Cette réaction s'effectue en deux étapes :
- a) 1ère étape : fabrication de l'ADNc par action de la réverse transcriptase à 42°C durant 13 minutes,
- b) 2ème étape : amplification génique classique: on chauffe à 95°C durant 3 minutes pour détruire la réverse-transcriptase et permettre l'étape de déshybridation/hybridation, puis on démarre le cycle décrit précédemment pour l'amplification génique.

L'invention a plus particulièrement pour objet une méthode de diagnostic in-vitro telle que décrite ci-dessus, dans laquelle l'étape de dénaturation est réalisée en présence du (ou des) couple(s) d'amorces (ou de primers) de l'invention. En effet, comme il a été précisé ci-dessus, une des caractéristiques des oligonucléotides (ou primers), de l'invention est de donner une bande d'amplification nette, dépourvue généralement de bandes aspécifiques, lorsqu'ils sont utilisés dans les conditions qui suivent :
- hybridation : les primers (1µl d'une solution à 40 µmolaire (40 µM) de chaque primer) sont mis en présence de l'ADN-matrice (100 à 300 ng) pour la première étape de dénaturation-réassociation ; on chauffe, durant 10 minutes à 100°C puis on plonge les tubes contenant ce mélange d'ADN-matrice et de primers dans de l'eau contenant de la glace afin d'augmenter le taux de réassociation ADN-matrice/primers. Les primers doivent être utilisés à une concentration finale dans l'étape d'amplification qui suit de 0,8 µM chaque.
- amplification : on ajoute au milieu précédent les 4 A dNTP chacun étant utilisé à 0,5 µmolaire en solution finale (50 µl), et une unité de Taq-polymérase pour un milieu réactionnel de 50 µl ; cette étape est réalisée dans un tampon d'amplification de la présente invention, généralement désigné sous le nom de "10 X buffer" dont la composition (lorsqu'il est dilué au 1/10°) est la suivante : Tris-HCl, pH 8,9 : 50 mM ; (NH₄)₂ SO₄ : 15 mM ; MgCl2 : 5 mM ; β-mercapto-éthanol : 10 mM ; gélatine : 0,25 mg/ml. On additionne 5 µl de ce tampon et de l'eau q.s.p. 50 µl au milieu précédent.

Les cycles d'amplification sont réalisés de la manière suivante : 30 à 40 cycles composés de :
. 94°C durant 10 secondes (dénaturation),
. 60°C durant 1 minute 30 (hybridation),
. 78°C durant 1 minute 30 (élongation).

Le tout sera suivi par un cycle unique à 78°C durant 15 minutes.

La précision des températures indiquées à ± 0,3°C près, ainsi que leur stabilité durant les différents cycles, représentent des conditions essentielles pour l'obtention des rendements maximum ainsi que l'absence de bandes aspécifiques.

La concentration optimale d'ADN est de 100 à 300 ng pour de l'ADN génomique extrait de cellules (de patients ou en culture, de mammifères ou autres).

Il va de soi que les conditions qui précèdent représentent des conditions optimales pour un milieu réactionnel final de 50 µl, et que ces conditions peuvent être modifiées en fonction du volume final du milieu réactionnel.

L'utilisation de plusieurs couples d'amorces différents (ou coktails de couples) de l'invention permet soit la détection croisée de plusieurs types de virus du type HIV et/ou SIV, soit la détection simultanée de plusieurs gènes d'un même virus du type HIV et/ou SIV.

A titre d'exemple de couples d'amorces préférés utilisables dans le cadre de la présente invention, on peut citer les couples d'amorces suivants :
- MMy1-MMy4, MMy2-MMy4, MMy1-MMy3, MMy18-MMy19, MMy4bis-MMy28bis, MMy28-MMy29bis, MMy29-MMy30bis, MMy31-MMy32bis, notamment pour le diagnostic in vitro de l'infection d'un individu par HIV-1 et/ou HIV-2
- MMy5-MMy8, MMy6-MMy8, MMy7-MMy8, MMy5-MMy7bis, MMy6-MMy7bis, MMy9-MMy11, MMy10-My11, MMy9-MMy10bis, MMy26-MMy5bis, MMy8bis-MMy9bis, MMy8bis-MMy89, MMy89bis-MMy9bis, MMy15-MMy17, MMy15-MMy16bis, MMy16-MMy17, MMy25-MMy27, MMy26-MMy27, notamment pour le diagnostic in vitro de l'infection d'un individu par HIV-1,
- MMy20-MMy22, MMy20-MMy2lbis, MMy21-MMy22, MMy23-MMy24, MMy12-MMy14, MMy12-MMy13bis, pour le diagnostic in vitro de l'infection d'un individu par HIV-2.

L'agent de polymérisation utilisé dans l'étape d'élongation du cycle est une ADN polymérase thermostable, notamment la Taq polymérase, l'amplifiose de la firme Appligène ou toute ADN-polymérase thermostable pouvant être commercialisée.

D'une manière générale, le cycle de la méthode de diagnostic in vitro de l'invention est répété entre 30 et 40 fois.

La méthode de diagnostic in vitro de l'invention permet également, en fonction des couples d'amorces nucléotidiques utilisés, de détecter sélectivement les gènes des virus du type HIV et/ou SIV présents dans l'échantillon biologique.

Les couples d'amorces utilisables, à titre d'exemples, pour la méthode de diagnostic gène par gène sus-mentionnée de l'invention, sont les suivants :
- MMy1-MMy4, MMy2-MMy4, MMy1-MMy3, MMy4bis-MMy28bis pour le gène gag,
- MMy18-MMy19 pour le gène vpr,
- MMy5-MMy8, MMy6-MMy8, MMy7-MMy8, MMy5-MMy7bis, MMy6-MMy7bis, MMy26-MMy5bis, MMy8bis-MMy9bis, MMy8bis-MMy89, MMy89bis-MMy9bis pour le gène env,
- MMy9-MMy11, MMy9-MMy10bis, MMy10-MMy11 pour le gène nefl,
- MMy15-MMy17, MMy15-MMy16bis, MMy16-MMy17, pour le gène vif1,
- MMy20-MMy22, MMy20-MMy21bis, MMy21-MMy22 pour vif 2,
- MMy23-MMy24 pour vpx,
- MMy12-MMy14, MMy12-MMy13bis, MMy13-MMy14 pour nef2,
- MMy25-MMy27, MMY26-MMy27 pour le gène vpu,
- MMy28-MMy29bis, MMy29-MMy30bis, MMy30-MMy31bis, 1-MMy32bis pour le gène pol.

Toutefois, les combinaisons entre primers "S" et "AS" décrites ci-dessus ne sont pas limitatives et peuvent être variées selon le désir de l'utilisateur.

Les tailles des fragments nucléotidiques synthétisés à l'aide des couples d'amorces susmentionnés à titre d'exemples, sont indiquées sur les tableaux I à XI suivants :
(les chiffres indiqués dans les tableaux ci-dessous représentent le nombre de nucléotides des fragments synthétisés, et les "tirets" indiquent que les couples d'amorces testés ne permettent pas de caractériser les souches virales correspondantes).

**Tableau I**

| | gag | | gag | |
|---|---|---|---|---|
| | MMy1-MMy3 | MMy1-MMy4 | MMy2-MMy4 | MMy4bis-MMy28bis |
| HIV1-BRU | 265 | 750 | 532 | 671 |
| HIV1-MAL | 282 | 785 | 556 | 671 |
| HIV1-ELI | 265 | 750 | 538 | 674 |
| HIV2-ROD | 354 | 845 | 544 | 663 |
| SIV | 343 | 844 | 544 | 668 |

**Tableau II**

| | env | | env | |
|---|---|---|---|---|
| | MMy5-MMy7bis | MMy5-MMy8 | MMy6-MMy7bis | MMy6-MMy8 |
| HIV1-BRU | 480 | 953 | 330 | 803 |
| HIV1-MAL | 471 | 944 | 321 | 794 |
| HIV1-ELI | 471 | 941 | 321 | 791 |
| HIV2-ROD | - | - | - | - |
| SIV | - | - | - | - |

**Tableau III**

| | env | | env |
|---|---|---|---|
| | MMy7-MMy8 | MMy26-MMy5bis | MMy8bis-MMy9bis |
| HIV1-BRU | 498 | 691 | 1038 |
| HIV1-MAL | 498 | 691 | 1041 |
| HIV1-ELI | 495 | 679 | 1038 |
| HIV2-ROD | - | - | - |
| SIV | - | - | - |

**Tableau IV**

| | env | env |
|---|---|---|
| | MMy8bis-MMy89 | MMy89bis-MMy9bis |
| HIV1-BRU | 411 | 646 |
| HIV1-MAL | 411 | 649 |
| HIV1-ELI | 411 | 646 |
| HIV2-ROD | - | - |
| SIV | - | - |

**Tableau V**

| | nef1 | nef1 | |
|---|---|---|---|
| | MMy9-MMy10bis | MMy9-MMy11: | MMy10-MMy11 |
| HIV1-BRU | 293 | 660 | 388 |
| HIV1-MAL | 302 | 660 | 388 |
| HIV1-ELI | 296 | 663 | 388 |
| HIV2-ROD | - | - | - |
| SIV | - | - | - |

**Tableau VI**

| | nef2 | | nef2 |
|---|---|---|---|
| | MMy12-MMy13bis | MMy12-MMy14 | MMy13-MMy14 |
| HIV1-BRU | - | - | - |
| HIV1-MAL | - | - | - |
| HIV1-ELI | - | - | - |
| HIV2-ROD | 400 | 792 | 415 |
| SIV | 400 | 755 | 378 |

**Tableau VII**

| | vif1 | | vif1 |
|---|---|---|---|
| | MMy15-MMy16bis | MMy15-MMy17 | MMy16-MMy17 |
| HIV1-BRU | 333 | 603 | 293 |
| HIV1-MAL | 333 | 603 | 293 |
| HIV1-ELI | 333 | 603 | 293 |
| HIV2-ROD | - | - | - |
| SIV | - | - | - |

**Tableau VIII**

| | vpr | vif2 | |
|---|---|---|---|
| | MMy18-MMy19: | MMy20-MMy21bis | MMy20-MMy22 |
| HIV1-BRU | 281 | - | - |
| HIV1-MAL | 281 | - | - |
| HIV1-ELI | 281 | - | - |
| HIV2-ROD | 319 | 352 | 659 |
| SIV | 308 | 352 | 656 |

**Tableau IX**

| | vif2 | vpx |
|---|---|---|
| | MMy21-MMy22 | : MMy23-MMy24 |
| HIV1-BRU | - | - |
| HIV1-MAL | - | - |
| HIV1-ELI | - | - |
| HIV2-ROD | 329 | 329 |
| SIV | 326 | 329 |

**Tableau X**

| | vpu | | pol |
|---|---|---|---|
| | MMy25-MMy27 | MMy26-MMy27 | MMy28-MMy29bis |
| HIV1-BRU | 263 | 104 | 623 |
| HIV1-MAL | 263 | 101 | 584 |
| HIV1-ELI | 263 | 101 | 584 |
| HIV2-ROD | - | - | 666 |
| SIV | - | - | 712 |

**Tableau XI**

| | pol | | pol |
|---|---|---|---|
| | MMy29-MMy30bis | MMy30-MMy31bis | MMy31-MMy32bis |
| HIV1-BRU | 742 | 869 | 826 |
| HIV1-MAL | 742 | 869 | 826 |
| HIV1-ELI | 742 | 869 | 826 |
| HIV2-ROD | 742 | 866 | 826 |
| SIV | 742 | 866 | 826 |

Il est à noter que grâce à leur disposition sur le génome, les primers servant à l'amplification peuvent être combinés de telle façon qu'ils peuvent être utilisés comme sonde, soit après marquage au ³²p par kination, soit pour l'utilisation dans la technique des sondes froides pour vérifier la spécificité de la bande d'amplification observée lors d'une analyse par "Southern transfert". En plus de la combinaison classique des amorces pour qu'un troisième oligonucléotide puisse servir de sonde interne spécifique, il est à noter le cas particulier des gènes vif1/vpr et vif2/vpx dû au chevauchement de ces gènes, ce qui permet une détection croisée. En outre, lors d'une analyse par séquençage de l'ADN amplifié, ces oligonucléotides peuvent être utilisés comme amorce spécifique pour la DNA-polymérase permettant un double séquençage dans chaque sens, donc une double lecture des séquences, levant ainsi des ambiguïtés éventuelles d'interprétation.

L'invention a également pour objet les amorces, telles que définies ci-dessus, marquées, notamment de manière radioactive ou enzymatique, ainsi que leur utilisation en tant que sondes nucléotidiques, notamment dans le cadre de la méthode de diagnostic in vitro telle que décrite ci-dessus.

L'invention a également pour objet des oligonucléotides tels que décrits ci-dessus et comportant des sucres en conformation α. De tels oligonucléotides présentent la caractéristique d'inverser le sens de la double hélice formée avec la matrice (brin du génome du virus), cette double hélice passant ainsi de l'état "S" à l'état "AS".

L'invention concerne aussi les oligonucléotides décrits ci-dessus dont certains nucléotides sont méthylés et/ou comportent un ou plusieurs atomes de soufre notamment sur les adénines. De tels oligonucléotides présentent la caractéristique d'augmenter la stabilité de la double hélice, et par conséquent de mieux s'hybrider avec le brin d'ADN à amplifier.

L'invention concerne également les oligonucléotides tels que décrits ci-dessus et se présentant sous la forme dite "de bases modifiées" comportant des nucléotides sur lesquels sont greffés de façon covalente des agents chromophores (molécules aromatiques planes telles que l'acridine orange), notamment selon la méthode décrite dans l'article de C. Hélène paru dans "la Vie des Sciences", compte-rendus, série générale, tome 4, n^{o}1, p. 17-37. De tels oligonucléotides présentent la caractéristique d'être facilement détectables, notamment par fluorescence.

Les oligonucléotides de l'invention sont également utilisables pour la mise en oeuvre d'une méthode de diagnostic in vitro de l'infection de singes (macaque, singe de mangabeys ou singe vert) par le virus du type SIV, cette méthode reprenant les principales caractéristiques de celle décrite ci-dessus.

L'invention a également pour objet des kits de diagnostic pour la mise en oeuvre des méthodes de diagnostic in vitro sus-mentionnées. A titre d'exemple, un kit de diagnostic de la présente invention comprend :
- au moins un couple d'amorces oligonucléotidiques selon l'invention, chaque couple comprenant une amorce s'hybridant à l'un des brins de la séquence d'acide nucléique à détecter, et une amorce s'hybridant avec le brin complémentaire de ce dernier dans les conditions définies ci-dessus,
- des réactifs appropriés à la mise en oeuvre du cycle d'opérations d'amplification, notamment de l'ADN polymérase, et quatre nucléotides triphosphate différents, et le milieu réactionnel dénommé "10 X buffer" décrit ci-dessus.
- une (ou plusieurs) sonde pouvant être marquée, notamment par radioactivité ou par la technique des sondes froides, capable de s'hybrider spécifiquement avec la (ou les) séquence(s) d'acides nucléiques amplifiée(s) à détecter.

L'invention concerne aussi l'utilisation des amorces de l'invention indiquées ci-dessus pour la mise en oeuvre d'un procédé de synthèse de protéines codées par les séquences nucléotidiques amplifiées à l'aide de ces amorces.

A titre illustratif, ce procédé de synthèse de protéines comprend l'amplification de séquences nucléotidiques des génomes des virus du type HIV ou SIV (codant pour une protéine déterminée et ayant subi, le cas échant, certaines modifications de leurs nucléotides) par mise en contact desdites séquences avec au moins un couple d'amorces selon l'invention dans les conditions décrites ci-dessus, suivie de la traduction de ces séquences ainsi amplifiées en protéines ; cette dernière étape est réalisée notamment par transformation de cellules hôtes appropiées à l'aide de vecteurs contenant lesdites séquences amplifiées, et récupération des protéines produites dans ces cellules hôtes.

L'invention concerne également les polypeptides issus de la traduction des séquences (ou amorces) nucléotidiques de l'invention.

L'invention a également pour objet l'utilisation des amorces oligonucléotidiques anti-sens en tant qu'agents antiviraux en général, notamment dans la lutte contre le SIDA, ainsi que des compositions pharmaceutiques contenant ces amorces anti-sens en association avec un véhicule pharmaceutiquement acceptable.

L'invention concerne également les compositions immunogènes comprenant un ou plusieurs produits de traduction des séquences nucléotidiques selon l'invention, et/ou un ou plusieurs produits de traduction des séquences nucléotidiques amplifiées selon les procédés décrits ci-dessus à partir des amorces définies selon l'invention, ces produits de traduction étant associés à un véhicule pharmaceutiquement acceptable.

L'invention concerne les anticorps dirigés contre l'un ou plusieurs des produits de traduction décrits ci-dessus (ou en d'autres termes, susceptibles de former une réaction immunologique avec un ou plusieurs produits de traduction des séquences nucléotidiques selon l'invention, ou encore un ou plusieurs produits de traduction des séquences nucléotidiques amplifiées à partir des amorces définies selon l'invention) et leur utilisation pour la mise en oeuvre de méthodes de diagnostic in vitro de l'infection d'un individu par un virus du type HIV-1 et/ou HIV-2, ou d'un animal par au moins l'un des trois virus (HIV-1, HIV-2, SIV) selon les procédés connus de l'homme de l'Art.

A titre illustratif, une telle méthode de diagnostic in vitro selon l'invention comprend la mise en contact d'un échantillon biologique (notamment de sérum) prélevé chez un patient à l'étude, avec des anticorps selon l'invention, et la détection à l'aide de tout procédé approprié (notamment à l'aide d'anti-immunoglobulines marquées) des complexes immunologiques formés entre les antigènes des virus du type HIV ou SIV éventuellement présents dans l'échantillon biologique et lesdits anticorps.

L'invention a également pour objet des kits de diagnostic in vitro comprenant des anticorps selon l'invention et, le cas échéant, des réactifs appropriés à la mise en évidence de la réaction immunologique formée entre lesdits anticorps et les antigènes des virus HIV ou SIV.

L'invention vise également un procédé de préparation des polypeptides mentionnés ci-dessus, notamment ceux correspondant selon le code génétique universel aux séquences (ou amorces) nculéotidiques décrites ci-dessus, ce procédé étant caractérisé en ce que, partant de préférence de l'aminoacide C-terminal, l'on condense successivement deux à deux les aminoacyles successifs dans l'ordre requis, ou des aminoacyles et des fragments préalablement formés et contenant déjà plusieurs résidus aminoacyles dans l'ordre approprié, ou encore plusieurs fragments préalablement ainsi préparés, étant entendu que l'on aura eu soin de protéger au préalable toutes les fonctions réactives portées par ces aminoacyles ou fragment à l'exception des fonctions amine de l'un et carboxyle de l'autre ou vice-versa, qui doivent normalement intervenir dans la formation des liaisons peptidiques, notamment après activation de la fonction carboxyle, selon les méthodes connues dans la synthèse des peptides et ainsi de suite, de proche en proche, jusqu'à l'acide aminé N-terminal.

Par exemple, on aura recours à la technique de synthèse peptidique en solution homogène décrit par Houben Weyl dans "Methode der organischen Chemie" (Méthode de la Chimie organique) édité par E. Wunsch, vol. 15-I et II, THIEME, Stuttgart 1974, ou à celle de synthèse peptidique en phase solide décrite par R.D. Merrifield dans "Solid phase peptide synthesis" (J. Am. Chem. Soc., 45, 2149-2154).

L'invention concerne également un procédé de préparation des séquences (ou amorces) nucléotidiques décrites ci-dessus, ce procédé comprenant les étapes suivantes :
- incubation de l'ADN génomique, isolé à partir d'un des virus du type HIV ou SIV sus-mentionnés, avec de l'ADNase I, puis addition d'EDTA et purification par extraction au mélange phenol/chloroforme/alcool isoamylique (25/24/ 1) puis par l'éther,
- traitement de l'ADN ainsi extrait par de l'Eco R1 méthylase en présence de DTT, et purification par extraction telle que décrite ci-dessus,
- incubation de l'ADN ainsi purifié avec les 4 désoxynucléotides triphosphates dATP, dCTP, dGTP, et dTTP en présence de T4 ADN polymérase et d'ADN ligase de E. coli, puis purification selon la méthode décrite ci-dessus,
- le clonage des acides nucléiques ainsi obtenus dans un vecteur approprié et la récupération de l'acide nucléique recherché à l'aide d'une sonde appropriée.

Un procédé de préparation particulièrement avantageux des séquences nucléotidiques de l'invention comprend les étapes suivantes :
- la synthèse d'ADN en utilisant la méthode automatisée des β-cyanethyl phosphoramidite décrite dans Bioorganic Chemistry 4; 274-325 (1986),
- le clonage des acides nucléiques ainsi obtenus dans un vecteur approprié et la récupération de l'acide nucléique par hybridation avec une sonde appropriée.

Un autre procédé de préparation des séquences nucléotidiques de l'invention comprend les étapes suivantes :
- l'assemblage d'oligonucléotides synthétisés chimiquement, pourvus à leurs extrémités de sites de restriction différents, dont les séquences sont compatibles avec l'enchaînement en acides aminés du polypeptide naturel selon le principe décrit dans Proc. Natl. Acad. Sci. USA, 80; 7461-7465, (1983),
- le clonage des acides nucléiques ainsi obtenus dans un vecteur approprié et la récupération de l'acide nucléique recherché par hybridation avec une sonde appropriée.

## Revendications

1. Oligonucléotide utilisable en tant qu'amorce pour l'amplification de séquences d'acide nucléique, caractérisé en ce que sa séquence est choisie parmi les séquences conservées et spécifiques des gènes nef1, vif1 des virus HIV-1 Bru, HIV-1 Mal, et HIV-1 Eli, ou parmi les séquences complémentaires de ces dernières.

2. Oligonucléotide selon la revendication 1, caractérisé en ce qu'il contient une séquence conservée et spécifique d'un gène choisi parmi les gènes nef1, vif1 des virus HIV-1 Bru, ou HIV-1 Mal, ou HIV-1 Eli et des nucléotides supplémentaires, ou contient une séquence nucléotidique complémentaire de l'une de ces dernières séquences, étant entendu que les nucléotides supplémentaires éventuels qui "débordent" la séquence nucléotidique conservée spécifique du gène en question, du côté des extrémités 3' ou 5', coïncident de préférence avec ceux qui se trouvent placés en deçà des extrémités 3' ou 5' correspondant au sein même de la séquence complète des gènes des virus du type HIV-1 susmentionnés.

3. Oligonucléotide caractérisé en ce que sa séquence est modifiée par rapport à la séquence nucléotidique d'un oligonucléotide selon la revendication 1 ou 2, et en ce qu'elle s'hybride, à une température de 60°C ± 1°C susdites, avec ledit oligonucléotide selon la revendication 1 ou 2.

4. Oligonucléotide utilisable pour l'amplification de séquences d'acide nucléique, caractérisé en ce que sa séquence est issue du gène env des virus HIV-1 Bru, HIV-1 Mal et HIV-Eli, et en ce qu'elle répond à l'un des enchaînements nucléotidiques suivants :

MMy5 : CCA ATT CCC ATA CAT TAT TGT GCC CC
S,6905-6930,6903-6928,6860-6885

MMy5bis : GGG GCA CAA TAA TGT ATG GGA ATT GG
AS, 6930-6905, 6928-6903, 6885-6860,

MMy6 : AAT GGC AGT CTA GCA GAA GAA GA
S,7055-7077,7053-7075,7010-7032

MMy7 : ATC CTC AGG AGG GGA CCC AGA AAT T
S, 7360-7384, 7349-7373,7306-7330

MMy7bis : AAT TTC TGG GTC CCC TCC TGA GGA T
AS, 7384-7360,7373-7349,7330-7306

MMy8 : GTG CTT CCT GCT GCT CCC AAG AAC CC
AS, 7857-7832,7846-7821,7800-7775

MMy8bis : GGG TTC TTG GGA GCA GCA GGA AGC AC
S, 7832-7857, 7821-7846, 7775-7800,

MMy9 : ATG GGT GGC AAG TGG TCA AAA AGT AG
... ... ... ..A ... ... ... ... ..
S,8844-8869, 8836-8861, 8787-8812,

MMy9bis : CTA CTT TTT GAC CAC TTG CCA CCC AT
AS, 8869-8844, 8861-8836, 8812-8787,

MMy78 : TAT TAA CAA GAG ATG GTG G
S, 7629-7647, 7612-7630, 7572-7590,

MMy89 : CCA GCA AGA AAA GAA TGA A
S, 8224-8242, 8213-8231, 8167-8185,

MMy89bis : TTC ATT CTT TTC TTG CTG G
AS, 8242-8224, 8231-8213, 8185-8167.

5. Oligonucléotide caractérisé en ce que sa séquence hybride, à une température de 60°C ± 1°C avec une séquence selon la revendication 4.

6. Oligonucléotide selon la revendication 1, caractérisé en ce que sa séquence est issue du gène nef1 des virus HIV-1 Bru, HIV-1 Mal et HIV-Eli, répondant à l'un des enchaînements nucléotidiques suivants :

MMy10 : AAA AGA AAA GGG GGG ACT GGA
S, 9116-9136, 9117-9137, 9062-9082,

MMy10bis : TCC AGT CCC CCC TTT TCT TTT
AS,9136-9116, 9137-9117, 9082-9062,

MMy11 : AAA GTC CCC AGC GGA AAG TCC C
AS,9503-9483, 9505-9484, 9449-9428,

7. Oligonucléotide selon la revendication 1, caractérisé en ce que sa séquence est issue du gène vif 1 des virus HIV-1 Bru, HIV-1 Mal et HIV-Eli, répondant à l'un des enchaînements suivants :

MMy15 : GAT TAT GGA AAA CAG ATG GCA GGT GAT
S, 5073-5099, 5068-5094, 5037-5063,

MMy16 : GCA GAC CAA CTA ATT CAT CTG TA
S, 5383-5405, 5378-5400, 5347-5369,

MMy16bis : TAC AGA TGA ATT AGT TGG TCT GC
AS, 5405-5383, 5400-5378, 5369-5347,

MMy17 : CTT AAG CTC CTC TAA AAG CTC TA
AS, 5675-5653, 5670-5648, 5639-5617.

8. Couple d'amorces caractérisé en ce qu'il comprend des oligonucléotides selon l'une quelconque des revendications 1 à 7 associés selon l'une des possibilités suivantes : MMy7-MMy8, ou MMy9-MMy10bis, ou MMy15-MMy17, ou MMy8bis-MMy89 ou MMy89bis-MMy9bis.

9. Procédé d'amplification génique de séquences nucléiques de virus du type HIV-1 réalisé à partir d'un échantillon biologique, ce procédé comprenant principalement les étapes suivantes :
- une étape d'extraction de l'acide nucléique à détecter appartenant au génome du virus du type HIV-1 éventuellement présent dans l'échantillon biologique sus-mentionné, et, le cas échéant, une étape de traitement à l'aide d'une transcriptase inverse dudit acide nucléique si ce dernier est sous forme d'ARN,
- un cycle comprenant les étapes suivantes :
. dénaturation de l'acide nucléique double brin à détecter, ce qui conduit à la formation d'un acide nucléique simple brin,
. hybridation de chacun des brins d'acide nucléique, obtenus lors de l'étape de dénaturation précédente, avec au moins un oligonucléotide utilisé comme amorce, selon l'une des revendications 1 à 8, par mise en contact des brins sus-mentionnés avec au moins un couple d'amorces susmentionnées,
. formation à partir des amorces des ADN complémentaires aux brins sur lesquels elles sont hybridées en présence d'une ADN polymérase et de quatre nucléosides triphosphate (dNTP) différents, ce qui conduit à la formation d'un plus grand nombre d'acides nucléiques double brin à détecter qu'à l'étape de dénaturation précédente,
ce cycle étant répété un nombre de fois déterminé pour obtenir ladite séquence nucléique à détecter éventuellement présente dans l'échantillon biologique dans une proportion suffisante pour permettre sa détection,
- une étape de détection de la présence éventuelle de l'acide nucléique appartenant au génome du virus du type HIV-1 dans l'échantillon biologique.

10. Procédé selon la revendication 9, caractérisé en ce que l'étape d'extraction de l'ADN viral comprend les étapes suivantes :
. suspension du culot cellulaire dans 0,5 ml d'eau pyrolisée dans un Potter gros piston,
. broyage des cellules dit par "aller et retour",
. adjonction de Triton X100 pour 1 concentration finale de 0,1 %,
. dénaturation à la chaleur durant 15 à 25 minutes à 100°C,
. centrifugation courte pour n'éliminer que les débris cellulaires,
. précipitation de l'ADN durant la nuit à -20°C par adjonction de 2,5 volumes d'éthanol absolu et de 10 % du volume final d'acétate de sodium 3 Molaires.

11. Procédé selon la revendication 9, caractérisé en ce que l'étape de rétro-transcription de l'ARN viral comprend les étapes suivantes :
- 10 µg d'ARN extrait resuspendu dans de l'eau est mis en présence du couple d'amorces à la concentration de 0,8 µM chacun, dans un volume final de 40 µl, l'ensemble est dénaturé à 100°C durant 10 minutes puis plongé dans de l'eau glacée,
- l'on rajoute 10 µl du mélange suivant : 5 µl du tampon "10 X buffer" (comprenant lorsqu'il est dilué au 1/10° : Tis-HCl, pH = 8,9 : 50mM ; (NH₄)₂ SO₄ ; 15 mM ; MgCl₂ ; 5 mM ; β-mercapto-éthanol : 10 mM ; gélatine : 0,25 mg/ml) + 1 unité de reverse-transcriptase + 1 unité de Taq-polymérase + 1 µl du mélange des 4 dNTP à 25 mM chacun + de l'eau Q.S.P. 10 µl, la fabrication de l'ADNc se fait par action de la réverse transcriptase à 42°C durant 13 minutes, puis on chauffe à 95°C durant 3 minutes pour détruire la réverse-transcriptase.

12. Procédé selon l'une des revendications 9 à 11, caractérisé en ce que l'étape de dénaturation est réalisée en présence du (ou des) couple(s) d'amorces selon l'une quelconque des revendications 1 à 8.

13. Procédé selon la revendication 12, caractérisé en ce qu'il est réalisé dans les conditions suivantes :
- pour l'étape d'hybridation, les amorces (1 µl d'une solution à 40 µmolaire de chaque amorce) sont mises en présence de l'ADN-matrice (100 à 300 ng) pour la première étape de dénaturation-réassociation, puis on chauffe, durant 10 minutes à 100°C puis on plonge les tubes contenant ce mélange d'ADN-matrice et d'amorces dans de l'eau contenant de la glace, les amorces étant utilisées à une concentration finale dans l'étape d'amplification qui suit de 0,8 µM chaque ;
- pour l'étape d'amplification, on ajoute au milieu précédent les 4 dNTP chacun étant utilisé à 0,5 µmolaire en solution finale (50 µl), et une unité de Taq-polymérase pour un milieu réactionnel de 50 µl ; cette étape étant réalisée dans le tampon d'amplification désigné sous le nom de "10 X buffer" dont la composition est indiquée dans la revendication 11.

14. Application du procédé selon l'une quelconque des revendications 9 à 13 au diagnostic in vitro de l'infection d'un individu par un virus du type HIV-1 ou d'un animal par un virus du type HIV-1.

15. Procédé d'expression de protéines codées par les séquences nucléotidiques du virus du type HIV-1 amplifiables au moyen des oligonucléotides selon l'une quelconque des revendications 1 à 8 comprenant les étapes de
- mise en contact d'au moins un couple d'oligonucléotides spécifiques d'un gène choisi parmi les gènes env, nef1, vif1 d'un rétrovirus de type HIV-1, ces oligonucléotides répondant à des séquences selon l'une quelconque des revendications 1 à 8, avec des séquences nucléotidiques issues du génome d'un virus de type HIV-1, dans des conditions permettant l'hybridation de ces séquences avec les oligonucléotides,
- amplification des séquences nucléotidiques contenues dans des gènes env, nef1, vif1, de rétrovirus de type HIV-1, à partir des susdits oligonucléotides selon le procédé de l'une quelconque des revendications 9 à 13,
- récupération et traduction des séquences ainsi amplifiées pour obtenir de séquences de protéines.

16. Compositions immunogènes comprenant un (ou plusieurs) produit(s) de traduction des oligonucléotides répondant aux séquences nucléiques selon l'une quelconque des revendications 1 à 8, et/ou un (ou plusieurs) produit(s) de traduction des séquences nucléotidiques contenues dans l'un des gènes env, nef1, ou vif1 de rétrovirus du type HIV-1 amplifiées par le procédé selon l'une des revendications 9 à 13, après hybridation avec un couple d'oligonucléotides choisis parmi les oligonucléotides selon l'une quelconque des revendications 1 à 8, spécifiques d'un gène d'un virus du type HIV-1 choisi parmi les gènes env, nefl ou vifl et/ou un (ou plusieurs) produit(s) d'expression obtenus par le procédé selon la revendication 15.

17. Kit pour la mise en oeuvre d'une méthode selon l'une des revendications 9 à 13 comprenant :
- au moins un couple d'amorces oligonucléotidiques selon l'une quelconque des revendications 1 à 8,
- des réactifs appropriés à la mise en oeuvre du cycle d'opérations d'amplification, notamment de l'ADN polymérase, et quatre nucléotides triphosphate différents,
- le tampon "10 X buffer" tel que décrit dans la revendication 11,
- une (ou plusieurs) sonde(s), pouvant être marquée(s), capable(s) de s'hybrider avec la (ou les) séquence(s) d'acide nucléique amplifiée(s) à détecter.

18. Composition pour le traitement de maladies virales, notamment du SIDA, comprenant au moins une séquences nucléotidique anti-sens selon l'une des revendications 1 à 8 en association avec un véhicule pharmaceutiquement acceptable.

19. Anticorps susceptibles de former une réaction immunologique avec les produits de traduction des oligonucléotides répondant aux séquences nucléiques selon l'une des revendications 1 à 8, et/ou un (ou plusieurs) produit(s) de traduction des séquences nucléotidiques contenues dans l'un des gènes env, nef1 ou vif1 de virus du type HIV-1 amplifiées par la méthode selon l'une quelconque des revendications 9 à 13 après hybridation avec un quelconque oligonucléotide choisi parmi les oligonucléotides selon l'une quelconque des revendications 1 à 8, spécifiques d'un gène d'un virus du type HIV-1 choisi parmi les gènes env, nef1 ou vif1 et/ou un (ou plusieurs produit(s) d'expression obtenu(s) par le procédé selon la revendication 15.

20. Méthode de diagnostic in vitro de l'infection d'un individu ou d'un animal par un virus du type HIV-1 comprenant la mise en contact d'un échantillon biologique (notamment de sérum) prélevé chez un patient à l'étude, avec des anticorps selon la revendication 19, et la détection des complexes immunologiques formés entre les antigènes des virus du type HIV-1 éventuellement présents dans l'échantillon biologique et lesdits anticorps.

21. Kit pour la mise en oeuvre d'une méthode selon la revendication 20, comprenant des anticorps selon la revendication 19 et, le cas échéant, des réactifs appropriés pour la mise en évidence de la réaction immunologique formée entre lesdits anticorps et les antigènes des virus HIV-1.

22. Solution tampon ("10 x buffer") utilisable dans un procédé d'amplification de séquences d'acide nucléique au moyen d'oligonucléotides selon l'une quelconque des revendications 1 à 8, caractérisé en ce qu'il comprend, lorsqu'il est dilué en 1/10ème :
- Tris - Hcl, pH 8,9 ; 50 mM,
- (NH₄)₂SO₄ ; 15 mM,
- MgCl₂ ; 5 mM
- β-mercapto-éthanol ; 10 mM
- gélatine ; 0,25 mg/ml.

## Patentansprüche

1. Oligonucleotid, verwendbar als Starter (Aktivator) zur Amplifikation von Nucleinsäuresequenzen, dadurch gekennzeichnet, daß seine Sequenz unter den konservierten, spezifischen Sequenzen der Gene nef1, vif1 der Viren HIV-1 Bru, HIV-1 Mal und HIV-1 Eli ausgewählt wird oder unter den komplementären Sequenzen dieser letzteren.

2. Oligonucleotid nach Anspruch 1, dadurch gekennzeichnet, daß es eine konservierte und spezifische Sequenz eines Gens, das unter den Genen nefl, vif1 der Viren HIV-1 Bru oder HIV-1 Mal oder HIV-1 Eli und den ergänzenden Nucleotiden ausgewählt ist, oder eine komplementäre Nucleotidsequenz einer dieser letzteren Sequenzen enthält, wobei festgelegt ist, daß die möglichen ergänzenden Nucleotide, welche an der konservierten, spezifischen Nucleotidsequenz des fraglichen Gens an der 3'- oder 5'-Endung "überstehen", vorzugsweise mit jenen zusammenfallen, welche sich diesseits der 3'- oder 5'-Endungen angeordnet finden, welche dem Inneren selbst der vollständigen Sequenz der Gene der viren vom oben erwähnten HIV-Typ entsprechen.

3. Oligonucleotid, dadurch gekennzeichnet, daß seine Sequenz bezogen auf die Nucleotidsequenz eines Oligonucleotides nach Anspruch 1 oder 2 modifiziert ist und dadurch, daß es bei einer oben erwähnten Temperatur von 60°C ± 1°C mit dem Oligonucleotid gemäß Anspruch 1 oder 2 hybridisiert.

4. Oligonucleotid, verwendbar zur Amplifikation von Nucleinsäuresequenzen, dadurch gekennzeichnet, daß seine Sequenz vom Gen env der Viren HIV-1 Bru, HIV-1 Mal und HIV-Eli stammt und dadurch, daß es einer der folgenden Nucleotidketten entspricht:

HMy5 : CCA ATT CCC ATA CAT TAT TGT GCC CC
S,6905-6930,6903-6928,6860-6885

MMy5bis : GGG GCA CAA TAA TGT ATG GGA ATT GG
AS, 6930-6905, 6928-6903, 6885-6860,

MHy6 : AAT GGC AGT CTA GCA GAA GAA GA
S,7055-7077,7053-7075,7010-7032

HHy7 : ATC CTC AGG AGG GGA CCC AGA AAT T
S, 7360-7384, 7349-7373,7306-7330

MHy7bis : AAT TTC TGG GTC CCC TCC TGA GGA T
AS, 7384-7360,7373-7349,7330-7306

HMy8 : GTG CTT CCT GCT GCT CCC AAG AAC CC
AS, 7857-7832,7846-7821,7800-7775

HMy8bis : GGG TTC TTG GGA GCA GCA GGA AGC AC
S, 7832-7857, 7821-7846, 7775-7800,

HHy9 : ATG GGT GGC AAG TGG TCA AAA AGT AG
... ... ... ..A ... ... ... ... ..
S,8644-8869, 8836-8861, 8787-8812,

HMy9bis : CTA CTT TTT GAC CAC TTG CCA CCC AT
AS, 8869-8844, 8861-8836, 8812-8787,

HMY78 : TAT TAA CAA GAG ATG GTG G
S, 7629-7647, 7612-7630, 7572-7590,

HMy89 : CCA GCA AGA AAA GAA TGA A
S, 8224-8242, 8213-8231, 8167-8185,

MHy89bis : TTC ATT CTT TTC TTG CTG G
AS, 8242-8224, 8231-8213, 8185-8167.

5. Oligonucleotid, dadurch gekennzeichnet, daß seine Sequenz bei einer Temperatur von 60°C ± 1°C mit einer Sequenz nach Anspruch 4 hybridisiert.

6. Oligonucleotid nach Anspruch 1, dadurch gekennzeichnet, daß seine Sequenz vom Gen nef1 der Viren HIV-1 Bru, HIV-1 Mal und HIV-Eli stammt, entsprechend einer der folgenden Nucleotidketten:

HHy10 : AAA AGA AAA GGG GGG ACT GGA
S, 9116-9136, 9117-9137, 9062-9082,

MHy10bis : TCC AGT CCC CCC TTT TCT TTT
AS,9136-9116, 9137-9117, 9082-9062,

MHy11 : AAA GTC CCC AGC GGA AAG TCC C
AS,9503-9483, 9505-9484, 9449-9428,

7. Oligonucleotid nach Anspruch 1, dadurch gekennzeichnet, daß seine Sequenz vom Gen vif 1 der Viren HIV-1 Bru, HIV-1 Mal und HIV-1 Eli stammt, entsprechend einer der folgenden Ketten:

HHy15 : GAT TAT GGA AAA CAG ATG GCA GGT GAT
S, 5073-5099, 5068-5094, 5037-5063,

HHY16 : GCA GAC CAA CTA ATT CAT CTG TA
: S, 5383-5405, 5378-5400, 5347-5369,

MHy16bis : TAC AGA TGA ATT AGT TGG TCT GC
AS, 5405-5383, 5400-5378, 5369-5347,

MMy17 : CTT AAG CTC CTC TAA AAG CTC TA
AS, 5675-5653, 5670-5648, 5639-5617.

8. Starterpaar, dadurch gekennzeichnet, daß es Oligonucleotide gemäß einem der Ansprüche 1 bis 7 umfaßt, welche nach einer der folgenden Möglichkeiten verbunden sind: MMy7-MMy8 oder MMy9-MMy10bis oder MMy15-MMy17 oder NMy8bis-MMy89 oder MMy89bis-MMy9bis.

9. Verfahren zur Genamplifkation von Nucleinsäuresequenzen des Virus vom Typ HIV-1, welches ausgehend von einer biologischen Probe durchgeführt wird, wobei das Verfahren hauptsächlich die folgenden Stufen umfaßt:
- eine Stufe der Extraktion der nachzuweisenden Nucleinsäure, welche dem Genom des Virus vom Typ HIV-1 angehört und gegebenenfalls in der oben erwähnten biologischen Probe vorliegt und gegebenenfalls eine Stufe der Behandlung mittels einer inversen Transcriptase dieser Nucleinsäure, wenn die letztere in RNS-Form vorliegt,
- einen Zyklus, welcher die folgenden Stufen umfaßt:
. Denaturieren der nachzuweisenden doppelsträngigen Nucleinsäure, was zur Bildung einer einsträngigen Nucleinsäure führt,
. Hybridisieren jedes während der vorhergehenden Stufe der Denaturierung erhaltenen Nucleinsäurestranges mit wenigstens einem als Starter verwendeten Oligonucleotid nach einem der Ansprüche 1 bis 8, durch in Kontakt bringen der oben erwähnten Stränge mit wenigstens einem oben erwähnten Starterpaar,
. Bilden von DNS mit komplementären Strängen, ausgehend von den Startern, auf welchen sie in Gegenwart einer DNS-Polymerase und von vier verschiedenen Nucleosidtriphosphaten (dNTP) hybridisiert werden, was zur Bildung einer viel größeren Anzahl nachzuweisender doppelsträngiger Nucleinsäuren führt wie die vorhergehende Stufe der Denaturierung,
wobei dieser Zyklus eine Anzahl Male wiederholt wird, welche zum Erhalten der gegebenenfalls in der biologischen Probe vorliegenden, nachzuweisenden Nucleinsäure in einer zu ihrem Nachweis ausreichenden Menge, bestimmt wurde,
- eine Stufe des Nachweises der möglichen Gegenwart der Nucleinsäure, welche dem Genom des Virus vom Typ HIV-1 in der biologischen Probe angehört.

10. Verfahren nach Anspruch 9, dadurch gekennzeichnet, daß die Stufe der Extraktion von viraler DNS die folgenden Stufen umfaßt:
. Suspendieren des Zellrückstandes in 0,5 ml pyrolisiertem Wasser in einem Potter mit großem Kolben,
. Brechen der Zellen durch "Vor- und Rückgang",
. Zugabe von Triton X100 bis auf eine Endkonzentration von 0,1 %,
. Denaturieren unter Erhitzen während 15 bis 25 min bei 100°C,
. kurzes Zentrifugieren, um nur die Zellabfälle zu entfernen,
. Ausfällen von DNS über Nacht bei - 20°C durch Zugabe von 2,5 Volumenteilen absolutem Ethanol und auf 10 % des Endvolumens von 3-molarem Natriumacetat.

11. Verfahren nach Anspruch 9, dadurch gekennzeichnet, daß die Stufe der Retro-Transcription der viralen RNS die folgenden Stufen umfaßt:
- 10 µg in Wasser resuspendierte, extrahierte RNS wird mit dem Starter(Aktivator)paar jeweils in der Konzentration von 0,8 µm in einem Endvolumen von 40 µl in Kontakt gebracht, alles wird bei 100°C während 10 min denaturiert und dann in Eiswasser getaucht,
- man fügt 10 µl der folgenden Mischung zu: 5 µl Puffer "10 X Puffer" (welcher, wenn er auf 1/10 verdünnt ist, umfaßt: Tris-HCl, pH = 8,9: 50 mM; (NH₄)₂SO₄: 15 mM; MgCl₂: 5 mM; β-Mercaptoethanol: 10 mM; Gelatine: 0,25 mg/ml) + 1 Einheit reverse Transcriptase + 1 Einheit Taq-Polymerase + 1 µl der Mischung der 4 dNTP zu jeweils 25 mM + 10 µl Wasser Q.S.P., die Herstellung der cDNS geschieht durch Wirkung der reversen Transcriptase bei 42°C während 13 Minuten, dann erhitzt man auf 95°C während 3 min, um die reverse Transcriptase zu zerstören.

12. Verfahren nach einem der Ansprüche 9 bis 11, dadurch gekennzeichnet, daß die Denaturierungsstufe in Gegenwart von (einem) Starterpaar(en) nach einem der Ansprüche 1 bis 8 durchgeführt wird.

13. Verfahren nach Anspruch 12, dadurch gekennzeichnet, daß es unter den folgenden Bedingungen durchgeführt wird:
- für die Stufe der Hybridisierung werden die Starter (1 µl einer Losung zu 40 µMol jedes Starters) mit der DNS-Matrix (100 bis 300 mg) für die erste Stufe der Denaturierung-Wiederverbindung in Kontakt gebracht, dann erhitzt man für 10 min auf 100°C, dann taucht man die Röhrchen, welche diese Mischung aus DNS-Matrix und Starter enthalten, in Eis enthaltendes Wasser, wobei die Starter in einer Endkonzentration in der Amplifikationsstufe verwendet werden, welche jeweils 0,8 µM beträgt;
- für die Amplifikationsstufe fügt man dem vorhergehenden Milieu die 4 dNTP, wobei alle zu 0,5 µMol der Endlösung (50 µl) verwendet werden und eine Einheit Taq-Polymerase für ein Reaktionsmilieu von 50 µl zu; diese Stufe wird in als "10 X Puffer" bezeichnetem Amplifikationspuffer durchgeführt, dessen Zusammensetzung in Anspruch 11 angegeben ist.

14. Verwendung des Verfahrens nach einem der Ansprüche 9 bis 13 zur in vitro-Diagnose der Infektion einer Person durch einen Virus vom Typ HIV-1 oder eines Tieres durch einen Virus vom Typ HIV-1.

15. Verfahren zur Expression von Proteinen, welche durch die Nucleotidsequenzen des Virus vom Type HIV-1, amplifizierbar mittels der Oligonucleotide gemäß einem der Ansprüche 1 bis 8, codiert werden, umfassend die Stufen:
- in Kontakt bringen wenigstens eines Oligonucleotidpaares, das spezifisch für ein Gen ist, das unter den Genen env, nef1, vif1 eines Retrovirus vom Typ HiV-1 ausgewählt ist, wobei diese Oligonucleotide, welche Sequenzen gemäß einem der Ansprüche 1 bis 8 entsprechen, mit Nucleotidsequenzen, welche vom Genom eines Virus vom Typ HIV-1 stammen, unter Bedingungen, welche die Hybridisierung dieser Sequenzen mit den Oligonucleotiden erlauben,
- Amplifizieren der in den Genen env, nefl, vifl des Retrovirus vom Typ HIV-1 enthaltenen Nucleotidsequenzen ausgehend von den oben genannten Oligonucleotiden gemäß dem Verfahren nach einem der Ansprüche 9 bis 13,
- Gewinnen und Translatieren der so amplifizierten Sequenzen, um Proteinsequenzen zu erhalten.

16. Immunogene Zusammensetzung, umfassend ein (oder mehrere) Produkt(e) der Translation von Oligonucleotiden, entsprechend den Nucleinsäuresequenzen gemäß einem der Ansprüche 1 bis 8 und/oder ein (oder mehrere) Produkt(e) der Translation der Nucleotidsequenzen, welche in einem der Gene env, nefl oder vifl des Retrovirus vom Type HIV-1, amplifiziert durch das Verfahren nach einem der Ansprüche 9 bis 13, enthalten sind, nach Hybridisierung mit einem Oligonucleotidpaar, ausgewählt unter den Oligonucleotiden gemäß einem der Ansprüche 1 bis 8, welche spezifisch für ein Gen eines Virus vom Typ HIV-1, ausgewählt unter den Genen env, nefl oder vifl sind und/oder mit einem (oder mehreren) Expressionsrodukt(en), erhalten durch das Verfahren nach Anspruch 15.

17. Kit zur Durchführung eines Verfahrens nach einem der Ansprüche 9 bis 13, umfassend:
- klein wenigstens ein Oligonucleotid-Starterpaar gemäß einem der Ansprüche 1 bis 8,
- wenigstens ein Oligonucleotid-Starterpaar gemäß einem der Ansprüche 1 bis 8,
- geeignete Reagenzien zur Durchführung des Amplifikationsoperationscyclus, nämlich DNS-Polymerase und 4 verschiedene Nucleotidtriphosphate,
- den Puffer "10 X Puffer" wie er in Anspruch 11 beschrieben ist,
- eine (oder mehrere) Sonde(n), welche markiert werden kann bzw. können und mit der bzw. den nachzuweisenden amplifizierten Nucleinsäuresequenz(en) hybridisieren kann bzw. können.

18. Zusammensetzung für zur Behandlung viraler Erkrankungen, insbesondere von Aids, umfassend wenigstens eine Antisens-Nucleotidsequenz gemäß einem der Ansprüche 1 bis 8 in Verbindung mit einem pharmazeutisch annehmbaren Träger.

19. Antikörper, welche dazu geeignet sind, eine immunologische Reaktion mit den Produkten der Translation der Oligonucleotide, welche den Nucleinsäuresequenzen gemäß einem der Ansprüche 1 bis 8 entsprechen, und/oder einem (oder mehreren) Produkte(n) der Translation der Nucleotidsequenzen, welche in einem der Gene env, nefl oder vifl des Virus vom Typ-HIV-1 enthalten sind, welche durch das Verfahren gemäß einem der Ansprüche 9 bis 13 amplifiziert sind, einzugehen, nach Hybridisierung mit einem Oligonucleotid, das aus den Oligonucleotiden gemäß einem der Ansprüche 1 bis 8 ausgewählt ist, die spezifisch für ein Gen eines Virus vom Typ HIV-1 sind, welche aus den Genen env, nefl oder vifl ausgewählt sind und/oder mit einem (oder mehreren) Expressionsprodukt(en), erhalten durch das Verfahren nach Anspruch 15.

20. Verfahren zur in vitro-Diagnose der Infektion einer Person oder eines Tieres durch einen virus vom Typ HIV-1, umfassend das in Kontakt bringen einer biologischen Probe (nämlich Serum), welche einem zu untersuchenden Patienten entnommen wird, mit Antikörpern nach Anspruch 19 und den Nachweis des immunologischen Komplexes, welcher zwischen den Antigenen der Viren vom Typ HIV-1, welche gegebenenfalls in der biologischen Probe vorhanden sind, und den Antikörpern gebildet ist.

21. Kit zur Durchführung eines Verfahrens nach Anspruch 20, umfassend die Antikörper nach Anspruch 19, und gegebenenfalls geeignete Reagenzien zum Nachweis der immunologischen Reaktion, welche zwischen den Antikörpern und den Antigenen der HIV-1 Viren erzeugt wird.

22. Pufferlösung ("10 X Puffer"), verwendbar in einem Verfahren zum Amplifizieren von Nucleinsäuresequenzen mit Oligonucleotiden nach einem der Ansprüche 1 bis 8, dadurch gekennzeichnet, daß sie, wenn sie auf 1/10 verdünnt ist, umfaßt:
- Tris-HCl, pH 8,9: 50 mM,
- (NH₄)₂SO₄: 15 mM,
- MgCl₂: 5 mM
- β-Mercaptoethanol: 10 mM
- Gelatine: 0,25 mg/ml.

## Claims

1. Oligonucleotide, which can be used as primer for the amplification of nucleic acid sequences, characterized in that its sequence is chosen among sequences which are conserved and specific of the nef1, vif1 genes of HIV-1 Bru, HIV-1 Mal and HIV-1 Eli, or among complementary sequences of the latter sequences.

2. Oligonucleotide according to claim 1, characterized in that it contains a conserved and specific sequence of a gene chosen among the nef1, vif1 genes of HIV-1 Bru, HIV-1 Mal and HIV-1 Eli and additional sequences, or contains a nucleotide sequence complementary of one of these latter sequences, it being understood that the possible additional nucleotides which « extend over » the conserved, specific nucleotide sequence of the gene in question, on the side of the 3' or 5' ends, coincide preferably with those which are placed below the 3' or 5' ends corresponding to the very inside of the complete sequence of the genes of the HIV-1 type viruses mentioned above.

3. Oligonucleotide characterized in that its sequence is modified with respect to the nucleotide sequence of an oligonucleotide according to claim 1 or 2 and in that it hybridizes, at a temperature of 60°C ± 1°C, with said oligonucleotide according to claim 1 or 2.

4. Oligonucleotide which can be used for the amplification of nucleic acid sequences, characterized in that its sequence is derived from the env gene of HIV-1 Bru, HIV-1 Mal and HIV-1 Eli viruses and in that it replies to one of the following nucleotide chains.

MMy5 : CCA ATT CCC ATA CAT TAT TGT GCC CC
S,6905-6930,6903-6928,6860-6885

MMy5bis : GGG GCA CAA TAA TGT ATG GGA ATT GG
AS, 6930-6905, 6928-6903, 6885-6860,

MMy6 : AAT GGC AGT CTA GCA GAA GAA GA
S,7055-7077,7053-7075,7010-7032

MMy7 : ATC CTC AGG AGG GGA CCC AGA AAT T
S, 7360-7384, 7349-7373,7306-7330

MMy7bis : AAT TTC TGG GTC CCC TCC TGA GGA T
AS, 7384-7360,7373-7349,7330-7306

MMy8 : GTG CTT CCT GCT GCT CCC AAG AAC CC
AS, 7857-7832,7846-7821,7800-7775

MMy8bis : GGG TTC TTG GGA GCA GCA GGA AGC AC
S, 7832-7857, 7821-7846, 7775-7800,

MMy9 : ATG GGT GGC AAG TGG TCA AAA AGT AG
... ... ... ..A ... ... ... ... ..
S,8844-8869, 8836-8861, 8787-8812,

MMy9bis : CTA CTT TTT GAC CAC TTG CCA CCC AT
AS, 8869-8844, 8861-8836, 8812-8787,

MMy78 : TAT TAA CAA GAG ATG GTG G
S, 7629-7647, 7612-7630, 7572-7590,

MMy89 : CCA GCA AGA AAA GAA TGA A
S, 8224-8242, 8213-8231, 8167-8185,

MMy89bis : TTC ATT CTT TTC TTG CTG G
AS, 8242-8224, 8231-8213, 8185-8167.

5. Oligonucleotide, characterized in that its sequence hybridizes at a temperature of 60°C ± 1°C with a sequence according to claim 4.

6. Oligonucleotide according to Claim 1, characterized in that its sequence is derived from the nef1 gene of the HIV-1 Bru, HIV-1 Mal and HIV-Eli viruses, replying to one of the following nucleotide chains :

MMy10 : AAA AGA AAA GGG GGG ACT GGA
S, 9116-9136, 9117-9137, 9062-9082,

MMy10bis : TCC AGT CCC CCC TTT TCT TTT
AS,9136-9116, 9137-9117, 9082-9062,

MMy11 : AAA GTC CCC AGC GGA AAG TCC C
AS,9503-9483, 9505-9484, 9449-9428,

7. Oligonucleotide according to Claim 1, characterized in that its sequence is derived from the vif 1 gene of the HIV-1 Bru, HIV-1 Mal and HIV-Eli viruses, replying to one of the following nucleotide chains :

MMy15 : GAT TAT GGA AAA CAG ATG GCA GGT GAT
S, 5073-5099, 5068-5094, 5037-5063,

MMy16 : GCA GAC CAA CTA ATT CAT CTG TA
S, 5383-5405, 5378-5400, 5347-5369,

MMy16bis : TAC AGA TGA ATT AGT TGG TCT GC
AS, 5405-5383, 5400-5378, 5369-5347,

MMy17 : CTT AAG CTC CTC TAA AAG CTC TA
AS. 5675-5653, 5670-5648, 5639-5617.

8. Pair of oligonucleotide primers characterized in that it comprises oligonucleotides according to anyone of claims 1 to 7, in association according to one of the following possibilities: MMy7-MMy8, or MMy9-MMy10bis, or MMy15-MMy17, or MMy8bis-MMy89 or MMy89bis-MMy9bis.

9. Process for the gene amplification of nucleic sequences of HIV-1 type viruses, performed starting with a biological sample, this process comprising mainly the following steps :
- a step of extracting the nucleic acid to be detected, belonging to the genome of the HIV-1 type virus which may be present in the abovementioned biological sample, and, where appropriate, a step of treating, with a reverse transcriptase, the said nucleic acid if the latter is in RNA form,
- a cycle comprising the following steps :
. denaturation of the double stranded nucleic acid to be detected, which leads to the formation of a single-stranded nucleic acid,
. hybridization of each of the nucleic acid strands, obtained during the preceding denaturation step, with at least one oligonucleotide used as primer according to one of Claims 1 to 8, by bringing the abovementioned strands into contact with at least one pair of the abovementioned primers,
. formation, from the primers, of the DNAs complementary to the strands with which they are hybridized in the presence of a DNA polymerase and of four different nucleoside triphosphates (dNTP), leading to the formation of a greater number of double-stranded nucleic acids to be detected than at the preceding denaturation step,
this cycle being repeated a determined number of times in order to obtain the said nucleic sequence to be detected which may be present in the biological sample in a proportion sufficient to allow its detection,
- a step of detecting the possible presence of the nucleic acid belonging to the genome of the HIV-1 type virus in the biological sample.

10. Process according to Claim 9, characterized in that the step of extracting the viral DNA comprises the following steps :
. suspension of the cellular pellet in 0.5 ml of pyrolized water in a Potter apparatus with a large piston,
. grinding of the cells by a so-called « reciprocating movement »,
. addition of Triton X100 for a final concentration of 0.1%,
. heat denaturation for 15 to 25 minutes at 100°C,
. short centrifugation so as to remove only the cellular debris,
. precipitation of the DNA overnight at -20°C by addition of 2.5 volumes of absolute ethanol and 10% of the final volume of 3 molar sodium acetate.

11. Process according to Claim 9, characterized in that the step of retrotranscription of the viral RNA comprises the following steps :
. 10 µg of extracted RNA resuspended in water are exposed to the pair of primers at the concentration of 0.8 µM each, in a final volume of 40 µl, the whole mixtrue is denaturated at 100°C for 10 minutes and then immersed in ice-cold water,
. 10 µl of the following mixture are added : 5 µl of the « 10 X buffer » (comprising when it is diluted 1/10: Tris-HCl, pH = 8.9: 50 mM ; (NH₄)₂SO₄ ; 15 mM ; MgCl₂ ; 5 mM; β-mercaptoethanol: 10 mM ; gelatin : 0.25 mg/ml) + 1 unit of reverse transcriptase + 1 unit of Taq polymerase + 1 µl of the mixture of the 4 dNTPs at 25 mM each + water qs 10 µl, the production of the cDNA is obtained by the action of the reverse transcriptase at 42°C for 13 minutes, then the mixture is heated at 95°C for 3 minutes, in order to destroy the reverse transcriptase.

12. Process according to one of Claims 9 to 11, characterized in that the denaturation step is performed in the presence of the pair(s) of primers according to one of Claims 1 to 8.

13. Process according to Claim 12, characterized in that it is performed under the following conditions :
- hybridization : the primers (1 µl of a 40 µmolar solution of each primer) are exposed to the template DNA (100 to 300 ng) for the first denaturation-reassociation step ; the mixture is heated for 10 minutes at 100°C and then the tubes containing this mixture of template DNA and primers are immersed in water containing ice. The primers should be used at a final concentration, in the subsequent amplification step, of 0.8 µM each,
- amplification there are added to the preceding medium the 4 A dNTPs, each being used at 0.5 µmolar final solution (50 µl), and one unit of Taq polymerase for a reaction medium of 50 µl ; this step is performed in the amplification buffer designated by the name « 10 X buffer » whose composition is indicated in Claim 11.

14. Application of the process according to any one of Claims 9 to 13 to the in vitro diagnosis of the infection of an individual by an HIV-1 type virus, or of an animal by an HIV-1 type virus.

15. Process for the expression of proteins encoded by the nucleotide sequences of the HIV-1 type virus, amplifiable with the oligonucleotides of anyone of claims 1 to 8 comprising the steps of:
- contacting at least a pair of oligonucleotides specific for a gene chosen among the env, nef1, vif1 genes of a HIV-1 retrovirus type, these oligonucleotides replying to the sequences according to anyone of claims 1 to 8, with nucleotide sequences derived from the genome of the HIV-1 type virus, in conditions allowing hybridization of these sequences with the oligonucleotides,
- amplification of the nucleotide sequences contained in env, nef1, vif1, genes of HIV-1 type retroviruses starting from the above said oligonucleotides according to the process of anyone of claims 9 to 13,
- recovery and translation of the sequences thus amplified to obtain the sequences of the proteins.

16. Immunogenic compositions comprising one (or several) product(s) of the translation of oligonucleotides replying to the nucleic sequences according to anyone of claims 1 to 8, and/or one (or several) product(s) of translation of the nucleotide sequences contained in one of the env, nef1, or vif1 retrovirus of the HIV-1 type amplified by the process according to anyone of claims 9 to 13, after hybridization with a pair of oligonucleotides according to anyone of claims 1 to 8, specific of a gene of a virus of the HIV-1 type chosen among the env, nef1, or vif1 genes and/or one (or several) expression products obtained by the process according to claim 15.

17. Kit for carrying out a method according to one of Claims 9 to 13 comprising :
- a least one pair of oligonucleotide primers according to any one of claims 1 to 8,
- reagents appropriate for carrying out the cycle of amplification operations, especially DNA polymerase, and four different nucleotide triphosphates,
- the 10 x buffer as described in Claim 11,
- one (or more) probe(s), which may be labelled, capable of hybridizing with the amplified nucleic acid sequence(s) to be detected.

18. Composition for the treatment of viral diseases, especially of AIDS, comprising at least one antisense nucleotide sequence according to one of Claims 1 to 8 in association with a pharmaceutically acceptable vehicle.

19. Antibodies capable of forming an immunological reaction with the products of translation of the oligonucleotide replying to the nucleic sequences according to one of Claims 1 to 8, and/or one (or more) product(s) of translation of the nucleotide sequences contained in one of the env, nef1, or vif1 genes of the HIV-1 type virus amplified by the method according to one of Claims 9 to 13 after hybridization with anyone of the oligonucleotides of claims 1 to 8, specific of a gene of a virus of the HIV-1 type chosen among the env, nef1 or vif1 genes and/or one (or several) expression products obtained by the process according to claim 15.

20. Method for the in vitro diagnosis of the infection of an individual or of an animal by an HIV-1 type virus, comprising bringing a biological sample (especially serum), collected from a patient under study, into contact with antibodies according to Claim 19, and the detection of the immunological complexes formed between the antigens of the HIV type viruses which may be present in the biological sample and the said antibodies.

21. Kit for carrying out a method according to Claim 20, comprising antibodies according to Claim 19 and, where appropriate, reagents appropriate for revealing the immunological reaction formed between the said antibodies and the antigens of the HIV viruses.

22. Buffer solution (« 10 x buffer ») which can be used in a process for amplification according to anyone of Claims 1 to 8 characterized in that it comprises, when it is diluted 1/10 :
- Tris-HCI, pH 8.9 ; 50 mM ;
- (NH₄)₂SO₄ ; 15 mM,
- MgCl₂ ; 5 mM,
- β-mercaptoethanol ; 10 mM,
- gelatin ; 0.25 mg/ml.
